# EUROPEAN PATENT APPLICATION

(11) **EP 1 783 142 A1**
(43) Date of publication of application: **09.05.2007**
(21) Application number: 05760155.1
(22) Date of filing: 08.07.2005
(51) Int. Cl.: C07K 16/44, C12N 15/09, C12P 21/08, G01N 33/53

(54) **MOUSE MONOCLONAL ANTIBODY RECOGNIZING ACETYLLYSINE, LABELED ANTIBODY AND UTILIZATION OF THE SAME**

(30) Priority: 09.07.2004 JP 2004203648
(71) Applicant: Advanced Life Science Institute, Inc., Wako-shi, Saitama 3510112 (JP)
(72) Inventor: KOMATSU, Yasuhiko, Ageo-shi, Saitama 3620042 (JP); IWABATA, Hisako, Saitama-shi, Saitama 3300061 (JP)
(74) Representative: Denison, Christopher Marcus
(86) International application number: PCT/JP2005/013105
(87) International publication number: WO 2006/006695

(57) **Abstract**

A mouse monoclonal antibody whose light chain variable region amino acid sequence is specified as the sequence listed in SEQ ID NO: 2 or that sequence with 2 or 3 different amino acids, and the heavy chain variable region is specified by the sequence listed in SEQ ID NO: 4 or that sequence with 2 or 3 different amino acids.

SEQUENCE LISTING

<110> Advanced Life Science Institute, Inc.

<120> Acetyl lysine specific mouse monoclonal antibody

<130> R775

<160> 4

<170> PatentIn version 3.1

<210> 1
<211> 501
<212> DNA
<213> Human

<220>
<221> CDS
<222> (1)..(501)
<223>

<400> 1
gat gct gtg atg acc caa act cca ctc tcc ctg tct gtc agt ctt gga 48
Asp Ala Val Met Thr Gln Thr Pro Leu Ser Leu Ser Val Ser Leu Gly
1               5                   10                  15

gat caa gcc tcc atc tct tgc agg tct agt cag agc ctt gaa aac agt 96
Asp Gln Ala Ser Ile Ser Cys Arg Ser Ser Gln Ser Leu Glu Asn Ser
            20                  25                  30

aat gga aac acc tat ttg aac tgg tac ctc cag aaa cca ggc cag tct 144
Asn Gly Asn Thr Tyr Leu Asn Trp Tyr Leu Gln Lys Pro Gly Gln Ser
        35                  40                  45

cca cag ctc ctg atc tac agg gtt tcc aac cga ttt tct ggg gtc cta 192

Pro Gln Leu Leu Ile Tyr Arg Val Ser Asn Arg Phe Ser Gly Val Leu
    50                  55                  60

gac agg ttc agt ggt agt gga tca ggg aca gat ttc aca ctg aaa atc        240
Asp Arg Phe Ser Gly Ser Gly Ser Gly Thr Asp Phe Thr Leu Lys Ile
65                  70                  75                  80

agc aga gtg gag gct gag gat ttg gga gtt tat ttc tgc ctc caa gtt        288
Ser Arg Val Glu Ala Glu Asp Leu Gly Val Tyr Phe Cys Leu Gln Val
                85                  90                  95

aca cat gtc ccg tgg acg ttc ggt gga ggc acc aag ctg gaa atc aaa        336
Thr His Val Pro Trp Thr Phe Gly Gly Gly Thr Lys Leu Glu Ile Lys
            100                 105                 110

cgg gct gat gct gca cca act gta tcc atc ttc cca cca tcc agt gag        384
Arg Ala Asp Ala Ala Pro Thr Val Ser Ile Phe Pro Pro Ser Ser Glu
        115                 120                 125

cag tta aca tct gga ggt gcc tca gtc gtg tgc ttc ttg aac aac ttc        432
Gln Leu Thr Ser Gly Gly Ala Ser Val Val Cys Phe Leu Asn Asn Phe
    130                 135                 140

tac ccc aaa gac atc aat gtc aag tgg aag att gat ggc agt gaa cga        480
Tyr Pro Lys Asp Ile Asn Val Lys Trp Lys Ile Asp Gly Ser Glu Arg
145                 150                 155                 160

caa aat ggc gtc ctg aac agt                                            501
Gln Asn Gly Val Leu Asn Ser
                165

<210>  2
<211>  167
<212>  PRT
<213>  Human

<400> 2

Asp Ala Val Met Thr Gln Thr Pro Leu Ser Leu Ser Val Ser Leu Gly
1               5                   10                  15

Asp Gln Ala Ser Ile Ser Cys Arg Ser Ser Gln Ser Leu Glu Asn Ser
            20                  25                  30

Asn Gly Asn Thr Tyr Leu Asn Trp Tyr Leu Gln Lys Pro Gly Gln Ser
        35                  40                  45

Pro Gln Leu Leu Ile Tyr Arg Val Ser Asn Arg Phe Ser Gly Val Leu
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Leu Gln Val
                85                  90                  95

Thr His Val Pro Trp Thr Phe Gly Gly Gly Thr Lys Leu Glu Ile Lys
            100                 105                 110

Arg Ala Asp Ala Ala Pro Thr Val Ser Ile Phe Pro Pro Ser Ser Glu
        115                 120                 125

Gln Leu Thr Ser Gly Gly Ala Ser Val Val Cys Phe Leu Asn Asn Phe

130                    135                    140

Tyr Pro Lys Asp Ile Asn Val Lys Trp Lys Ile Asp Gly Ser Glu Arg
145                 150                 155                 160

Gln Asn Gly Val Leu Asn Ser
                165

<210>  3
<211>  498
<212>  DNA
<213>  Human

<220>
<221>  CDS
<222>  (1)..(498)
<223>

<400>  3
cag gtc caa ctg cag cag tct gga gct gag ctg gta agg cct ggg act        48
Gln Val Gln Leu Gln Gln Ser Gly Ala Glu Leu Val Arg Pro Gly Thr
1               5                   10                  15

tca gtg aag atg tcc tgc aag gct gct gga tac acc ttc act aac tac        96
Ser Val Lys Met Ser Cys Lys Ala Ala Gly Tyr Thr Phe Thr Asn Tyr
            20                  25                  30

tgg ata ggt tgg gta aag cag agg cct gga cat gac ctt gag tgg att       144
Trp Ile Gly Trp Val Lys Gln Arg Pro Gly His Asp Leu Glu Trp Ile
        35                  40                  45

gga gat att tac cct gga agt ggt tat gct tac tac aat gag aag ttc       192
Gly Asp Ile Tyr Pro Gly Ser Gly Tyr Ala Tyr Tyr Asn Glu Lys Phe

50                  55                  60

aag ggc aag gcc aca ctg act gca gac aca tcc tcc agc aca gcc tac        240
Lys Gly Lys Ala Thr Leu Thr Ala Asp Thr Ser Ser Ser Thr Ala Tyr
65                  70                  75                  80

atg cag ctc agc agc ctg aca tct gag gac tct gcc ttc tat tac tgt        288
Met Gln Leu Ser Ser Leu Thr Ser Glu Asp Ser Ala Phe Tyr Tyr Cys
                85                  90                  95

gta aga tcc tac ttc gct gat ggc ccg gcc tgg ttt gct tac tgg ggc        336
Val Arg Ser Tyr Phe Ala Asp Gly Pro Ala Trp Phe Ala Tyr Trp Gly
            100                 105                 110

caa ggg act ctg gtc act gtc tct gca gcc aaa acg aca ccc cca tct        384
Gln Gly Thr Leu Val Thr Val Ser Ala Ala Lys Thr Thr Pro Pro Ser
        115                 120                 125

gtc tat cca ctg gcc cct gga tct gct gcc caa act aac tcc atg gtg        432
Val Tyr Pro Leu Ala Pro Gly Ser Ala Ala Gln Thr Asn Ser Met Val
    130                 135                 140

acc ctg gga tgc ctg gtc aag ggc tat ttc cct gag cca gtg aca gtg        480
Thr Leu Gly Cys Leu Val Lys Gly Tyr Phe Pro Glu Pro Val Thr Val
145                 150                 155                 160

acc tgg aac tct gga tcc                                                498
Thr Trp Asn Ser Gly Ser
                165

<210>  4
<211>  166
<212>  PRT
<213>  Human

<400> 4

Gln Val Gln Leu Gln Gln Ser Gly Ala Glu Leu Val Arg Pro Gly Thr
1               5                   10                  15

Ser Val Lys Met Ser Cys Lys Ala Ala Gly Tyr Thr Phe Thr Asn Tyr
            20                  25                  30

Trp Ile Gly Trp Val Lys Gln Arg Pro Gly His Asp Leu Glu Trp Ile
        35                  40                  45

Gly Asp Ile Tyr Pro Gly Ser Gly Tyr Ala Tyr Tyr Asn Glu Lys Phe
    50                  55                  60

Lys Gly Lys Ala Thr Leu Thr Ala Asp Thr Ser Ser Ser Thr Ala Tyr
65                  70                  75                  80

Met Gln Leu Ser Ser Leu Thr Ser Glu Asp Ser Ala Phe Tyr Tyr Cys
                85                  90                  95

Val Arg Ser Tyr Phe Ala Asp Gly Pro Ala Trp Phe Ala Tyr Trp Gly
            100                 105                 110

Gln Gly Thr Leu Val Thr Val Ser Ala Ala Lys Thr Thr Pro Pro Ser
        115                 120                 125

Val Tyr Pro Leu Ala Pro Gly Ser Ala Ala Gln Thr Asn Ser Met Val
    130                 135                 140

Thr Leu Gly Cys Leu Val Lys Gly Tyr Phe Pro Glu Pro Val Thr Val
145                 150                 155                 160

Thr Trp Asn Ser Gly Ser
                165

## Description

### Technical Field

The present invention relates to an antibody that can be used to detect acetyllysine, which is a post-translational modification on proteins.

### Background Art

It is known that most proteins do not exhibit their function as their translated form per se, but instead variously post-translationally modified before exhibiting normal function (Int. J. Biochem. 24, 19-28, 1992). For example, protein phosphorylation is important as a signal cascade during transduction of extracellular signals to the nucleus or as a regulating factor to promote the normal cell cycle, while histone acetylation is important to efficiently promote transcription. Ubiquitinated proteins are carried to the proteasome where they are decomposed and lose activity, whereas most proteins are converted to their active forms via their signal peptides being clipped off by signal peptidases in the endoplasmic reticulum membrane. Thus, proteins are post-translationally modified in order to exhibit respective functions at appropriate times and locations.

Acetylation of lysine residues in proteins is a post-translational modification that was discovered in the 1960s. The first discovered lysine-acetylated protein was core histone (Proc. Natl. Acad. Sci. USA 51, 786-794, 1964). Although a relationship between acetylation and gene expression had been suspected at the time of the discovery, a detailed understanding thereof at the molecular level has been achieved only recently. Acetylation has been discovered for numerous proteins in addition to histone acetylation, suggesting that it is a post-translational modification of no less importance than phosphorylation (EMBO J. 19, 1176-1179, 2000). It is therefore believed that many heretofore unknown acetyllysine-containing proteins are yet to be discovered, several of which are potential target molecules for therapeutic agents and important molecules for diagnosis of disease, and hence it is desirable to develop means for efficiently identifying unknown acetylated proteins.

One effective method for this purpose involves using antibodies capable of recognizing acetyllysine residues independently of the amino acid sequences surrounding the acetyllysine residues. Such antibodies bind to unknown acetyllysine-containing proteins, thereby allowing efficient detection and purification of novel acetyllysine-containing proteins by methods such as immunological detection, affinity purification, immunoprecipitation and the like.

Several antibodies that are independent of adjacent sequences have either been marketed or published. An example of a marketed monoclonal antibody is the monoclonal antibody Ac-K-103 by Cell Signaling Tech. Also, Komatsu et al. performed immunization with Keyhole Limpet Hemocyanin (KLH) conjugated with an acetyllysine-containing peptide, or chemically acetylated KLH, to establish four different anti-acetyllysine mouse monoclonal antibodies (J Immunol. Methods 272, 161-175, 2003; WO02074962A1).

The sequences of the variable regions of all of these antibodies were found to converge on a single framework sequence, but the individual amino acid sequences were not completely identical. Anti-acetyllysine antibodies with even slightly superior detecting power are desired for detection of unknown acetyllysine-containing proteins, and it is possible that even antibodies with common frameworks having partial differences in their sequences become to have enhanced reactivity.
Patent document 1: WO02074962A1
Non-patent document 1: Int. J. Biochem. 24, 19-28, 1992
Non-patent document 2: Proc. Natl. Acad. Sci. USA 51, 786-794, 1964
Non-patent document 3: EMBO J. 19, 1176-1179, 2000
Non-patent document 4: J Immunol. Methods 272, 161-175, 2003

### Disclosure of the Invention

It is a problem of the present research to establish a more highly sensitive method than the prior art for recognizing acetyllysine residues in proteins, which method is minimally influenced by the type of protein or the types of amino acids surrounding the acetyllysine residues. It is a major problem of the present invention to obtain novel unlabeled or labeled anti-acetyllysine antibodies that can be used as probe molecules for such research.

As a result of much diligent research directed toward solving the problem stated above, the present inventors have discovered that it can be achieved by a labeled and unlabeled antibody having a specific variable region amino acid sequence. Specifically, the object is achieved by the following means.
(1) A mouse monoclonal antibody whose light chain variable region amino acid sequence is specified as the sequence listed as SEQ ID NO: 2 or as that sequence with 2 or 3 different amino acids, and the heavy chain variable region is specified by the sequence listed as SEQ ID NO: 4 or as that sequence with 2 or 3 different amino acids.
(2) A mouse monoclonal antibody according to (1) above, which recognizes Nε-acetyllysine.
(3) A mouse monoclonal antibody according to (1) above, wherein the light chain variable region amino acid sequence is specified as SEQ ID NO: 2, and the heavy chain variable region amino acid sequence is specified as SEQ ID NO: 4.
(4) A mouse monoclonal antibody according to (3) above, which recognizes Nε-acetyllysine.
(5) A monoclonal antibody according to (4) above, which is produced by a mouse hybridoma specified as deposit number FERM BP-10351.
(6) An anti-acetyllysine labeled antibody obtained by reducing an anti-acetyllysine antibody and binding a labeling molecule to the exposed sulfhydryl groups.
(7) An anti-acetyllysine labeled antibody according to (6) above, wherein the labeling molecule is horseradish peroxidase.
(8) An anti-acetyllysine labeled antibody according to (6) or (7), wherein the anti-acetyllysine antibody prior to labeling is an anti-acetyllysine antibody according to (2) above.
(9) An anti-acetyllysine labeled antibody according to (6) or (7), wherein the anti-acetyllysine antibody prior to labeling is an anti-acetyllysine antibody according to (4) above.
(10) An anti-acetyllysine labeled antibody according to (6) or (7), wherein the anti-acetyllysine antibody prior to labeling is an anti-acetyllysine antibody according to (5) above.
(11) An anti-acetyllysine labeled antibody according to any one of (7) to (10) above, wherein the method used to bind the labeling molecule to the anti-acetyllysine antibody is a method of binding to a portion other than an amino group of the antibody.
(12) An anti-acetyllysine labeled antibody according to (11) above, wherein the portion other than an amino group is a sulfhydryl group exposed by reduction of the antibody.

### Brief Explanation of the Drawings

Fig. 1 is a pair of graphs showing differences in the reactivities of ACK2F12 antibody and AKL5C1 antibody for (A) non-acetylated BSA and (B) acetylated BSA. Both antibodies reacted with acetylated BSA but not with non-acetylated BSA.
Fig. 2 shows the chemical structures of lysine, Nα-acetyllysine and Nε-acetyllysine.
Fig. 3 is a pair of graphs showing the effects of (A) Nα-acetyllysine and (B) Nε-acetyllysine on binding of ACK2F12 antibody and AKL5C1 antibody to acetylated BSA. Here it is demonstrated that binding of the antibodies is not competed with Nα-acetyllysine, and competed with Nε-acetyllysine.
Fig. 4 is a table showing the sequences of the acetyllysine-containing peptides and non-containing peptides used in the experiment of Fig. 5.
Fig. 5A is a graph showing the reactivity of anti-acetyllysine mouse monoclonal antibody AKL5C1 for different acetyllysine-containing peptides.
Fig. 5B is a graph showing the reactivity of anti-acetyllysine mouse monoclonal antibody ACK2F12 for different acetyllysine-containing peptides.
Fig. 5C is a graph showing the reactivity of anti-acetyllysine mouse monoclonal antibody Ac-K-103 (Cell Signaling Tech.) for different acetyllysine-containing peptides.
Fig. 5D is a graph showing the reactivity of anti-histone H3 (Ac-Lys-14) marketed by UpState Co. for different acetyllysine-containing peptides.
Fig. 5E is a graph showing the reactivity of anti-histone H4 (Ac-Lys-16) rabbit polyclonal antibody marketed by UpState Co. for different acetyllysine-containing peptides.

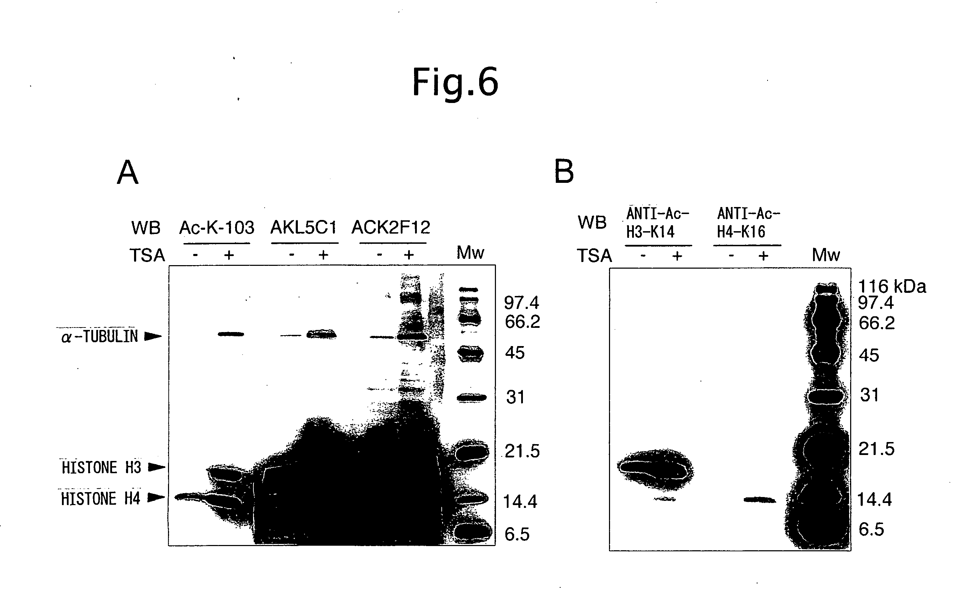

Fig. 6 is a pair of photographs showing a comparison of outputs by Western blotting for (A) three different anti-acetyllysine mouse monoclonal antibodies AKL5C1, ACK2F12 and Ac-K-103 (Cell Signaling Tech.) and (B) anti-histone H3 (Ac-Lys-14) and anti-histone H4 (Ac-Lys-16) rabbit polyclonal antibodies marketed by UpState Co. Lysate corresponding to 6 µg of protein was loaded in each lane. The primary antibody concentration was 1 µg/ml except for anti-histone H4 (Ac-Lys-16). For anti-histone H4 (Ac-Lys-16) the stock solution was used at 1000-fold dilution. Here it is seen that the greatest number of bands could be detected with ACK2F12 antibody.
Fig. 7 is a photograph showing a comparison of outputs by Western blotting for AKL5C1 and ACK2F12 antibodies using three different cultured cell lysates (MOLT-4F, HepG2 and 1B2C6) (30 µg/lane). Detection of more bands was possible with ACK2F12 antibody than with AKL5C1 antibody.
Fig. 8 is a schematic diagram showing the differences between two methods of labeling an antibody with horseradish peroxidase (HRP).
Fig. 9 is a pair of photographs comparing the results of (A) detection of acetylated protein in MOLT-4F cell lysate using Ac-K-103, AKL5C1 and ACK2F12 antibodies as primary antibodies without labeling and HRP-labeled anti-mouse IgG antibody as secondary antibody, after electrophoresis of trichostatin A-treated and -untreated MOLT-4F cell lysate (2 µg/lane) and transfer to a PVDF membrane, and (B) detection using ACK2F12 antibody directly labeled with HRP. It is seen that output was highest with SH group HRP-labeled ACK2F12 antibody.
Fig. 10 is a photograph showing the results of detection of chemically acetylated BSA by Western blotting using ACK2F12-HRP-SH labeled antibody, indicating that at least 80 pg of protein could be detected.
Fig. 11 is a photograph showing the results of detecting acetyllysine-containing protein in different mouse organs using ACK2F12-HRP-SH, where the organ lysates were developed by electrophoresis (30 µg/lane) and then transferred to a PVDF membrane.
Fig. 12 is a photograph showing the results for brain and stomach lysates that were developed by electrophoresis (15 µg/lane) and then transferred to a PVDF membrane, in the presence of 10 mM lysine, Nα-acetyllysine or Nε-acetyllysine during reaction with ACK2F12-HRP-SH. It is seen that antibody-dependent detection of bands was inhibited only in the presence of Nε-acetyllysine.
Fig. 13A shows the cDNA sequence coding for the light chain variable region of ACK2F12 antibody, aligned with the cDNA sequences coding for the variable regions of AL3D5, AL11, AKL3H6 and AKL5C1 antibodies. The matching portions are highlighted in black.
Fig. 13B shows the cDNA sequence coding for the heavy chain variable region of ACK2F12 antibody, aligned with the cDNA sequences coding for the heavy chain variable regions of AL3D5, AL11, AKL3H6 and AKL5C1 antibodies. The matching portions are highlighted in black.
Fig. 14 shows the amino acid sequence of the variable region of ACK2F12 antibody, aligned with the amino acid sequences of the variable region of AL3D5, AL11, AKL3H6 and AKL5C1 antibodies. (A) light chain, (B) heavy chain. The matching portions are highlighted in black.
Fig. 15 is a pair of tables listing the numbers of nucleotides or amino acids differing in the (A) cDNA sequences and (B) amino acid sequences for the variable regions of ACK2F12, AL3D5, AL11, AKL3H6 and AKL5C1 antibodies. The results at top right of the diagonal line are for the light chains, and those at the bottom left are for the heavy chains.
Fig. 16 lists the results of determining the amino acid sequences up to 10 residues from the N-terminus of the light and heavy chains of AL11, AL3D5, AKL3H6, AKLSC1, ACK2F12 and commercially available Ac-K-103 antibody, using a protein sequencer. Results were obtained for all of the light chains, but the sequences could not be determined for the heavy chains because PTH amino acid cleavage was unsuccessful.

### Best Mode for Carrying Out the Invention

The method of preparing the anti-acetyllysine antibody may be any method, and the antigen used for immunization may be, for example, a protein such as keyhole limpet hemocyanin (KLH) or bovine serum albumin (BSA) which has been chemically acetylated, or it may be a peptide containing an acetyllysine residue bonded to a protein such as KLH. As proteins to be practically applied for the purpose described above there may be mentioned, in addition to KLH and BSA, also ovalbumin, human serum albumin, transferrin, thyroglobulin and the like, although there is no limitation to these.

The amino acid sequence of the variable region of the antibody is specified according to the invention, and the amino acid sequence of the variable region of the antibody may be determined by the following method, for example. RNA is extracted from a monoclonal antibody-producing hybridoma and used as template for production of cDNA. Next, a primer set corresponding to the sections flanking the antibody variable region is used for PCR reaction to amplify cDNA coding for the variable region. The amplified cDNA may be directly subjected to a DNA sequencer to read the sequence, or it may be first inserted in a cloning vector and replicated in *E*. *coli* prior to sequence reading. The obtained cDNA sequence may be translated to the amino acid sequence to determine the amino acid sequence of the variable region. As a different method, the antibody molecule may be reduced to divide it into light and heavy chains, and then separated by electrophoretic or other means, and the respective amino acid sequences directly determined using means such as a gas-phase sequencer based on the principle of Edman degradation.

The means for detecting acetyllysine-containing protein may be a method such as Western blotting with antibody. Here, the established antibody may be used directly, with corresponding labeled anti-immunoglobulin antibody as the secondary antibody, but the present inventors have found that direct labeling of the antibody with a reporter enzyme allows significantly improved detection sensitivity. Several methods have been reported for labeling of antibodies with labeling molecules such as HRP, but it has been found that for anti-acetyllysine antibodies, the superior method is to utilize the sulfhydryl groups exposed by weak reduction of the antibodies, rather than using amino groups on the antibody molecules. In other words, for anti-acetyllysine antibodies it is preferred not to utilize binding of the amino groups of the antibodies with other molecules.

The present invention will now be explained in detail using the example of an anti-acetyllysine antibody produced by a hybridoma established using spleen cells of mice immunized using chemically acetylated Keyhole Limpet Hemocyanin (KLH), with the understanding that the working mode of the invention is not limited thereto.

The chemically acetylated KLH may be produced according to a reported method (J. Immunol. Methods 272, 161-175, 2003).

A method of immunizing animals will now be explained for mice. An acetylated KLH solution is mixed with an adjuvant such as Freund's complete adjuvant or TiterMax Gold (CytRx) at 1:1, and repeatedly passed through a joint comprising two syringes joined at a cross-flow joint or treated with ultrasonic waves, to form an emulsion. The prepared antigen-containing emulsion is then injected subcutaneously, intradermally, intramuscularly or intraperitoneally, or at multiple sites. The completed first immunization may be followed by booster immunizations in the same manner after a period of 1-4 weeks. When Freund's complete adjuvant is used for the first immunization, it is preferred to use Freund's incomplete adjuvant for the subsequent booster immunizations. Immunization is continued in the same manner until the blood antibody titer of anti-acetyllysine antibody increases.

Measurement of the antibody titer may be accomplished in the following manner. Bovine serum albumin acetylated by the same method as KLH is dissolved in PBS to a concentration of 10 µg/ml, and added to each well of a 96-well ELISA plate at a volume of 50 µl per well, for adsorption overnight at 4°C. Each well is washed with PBS containing 0.05% Tween 20 (PBST) and used for assay. Prior to the assay, blocking may be performed with PBST containing 1% BSA. Blood is sampled from the orbital venous plexus, the caudal vein, the caudal artery or the like, diluted 30-fold with PBST and then centrifuged. The obtained supernatant is prepared into a dilution series with PBST, and added at 50 µl to each well of an ELISA plate coated with the acetylated BSA. After reaction at room temperature for 30 minutes and rinsing with PBST, horseradish peroxidase (HRP)-labeled anti-mouse IgG solution appropriately diluted with PBST is added at 50 µl to each well. After further reaction at room temperature for 30 minutes, color is developed by adding a solution of an HRP substrate such as ortho-phenylenediamine.

Upon confirming sufficient titer increase, the spleen is removed and the spleen cells are isolated. These are fused with separately cultured mouse myeloma cells (for example, SP2/0-Ag14) using polyethylene glycol or the like. The successfully fused cells are selectively cultured in HAT (hypoxanthine/aminopterin/thymidine) medium. Half of the medium is exchanged every few days for about 7-14 days while culturing is continued, and then the antibody titer of the culture supernatant is measured. The positive well cells are cloned by the limiting dilution method to obtain hybridomas producing the target antibody. As a hybridoma clone obtained by this method there may be mentioned ACK2F12 (deposit number: FERM BP-10351). This clone was internationally deposited as FERM BP-10351 on June 18, 2004 at the National Institute of Advanced Industrial Science and Technology, International Patent Organism Depositary (IPOD) (Tsukuba Central 6, 1-1, Higashi 1-chome, Tsukuba-shi, Ibaraki-ken, Japan) (transferred from FERM P-20089 also deposited on June 18, 2004).

A method such as the following may be used to confirm that the antibody obtained by this method recognizes acetyllysine residues with low dependency on the surrounding amino acid sequence. First, recognition of acetyllysine residues by the antibody may be confirmed by determining that reactivity of the antibody for solid phased acetylated proteins is inhibited by externally added acetyllysine. Low surrounding amino acid sequence dependency can be judged by examining whether the antibody reacts with acetyllysine-containing peptides having different adjacent sequences. After electrophoresis of the solubilized animal cells, they are transferred to a PVDF membrane or nitrocellulose membrane and stained by Western blotting with antibody, whereby multiple proteins are stained.

Any method may be used for labeling of the antibody with a reporter molecule such as an enzyme or fluorescent dye, but preferably labeling of the anti-acetyllysine antibody does not utilize amino groups of the antibody molecule. Specifically, the antibody is lightly reduced with a reducing agent such as cysteamine to expose the sulfhydryl groups in the molecule, and then a labeling molecule activated with maleimide or the like is bonded thereto to label the antibody with a reporter molecule without utilizing amino groups of the antibody molecule.

It can be confirmed whether the prepared antibody and modified antibody are superior to conventional antibody by, for example, determining whether more acetyllysine-containing protein bands can be detected when the antibody or modified antibody is used at the same concentration.

The antibody and modified antibody provided by the present invention may be used not only to search for acetyllysine-containing proteins as new target molecules useful for diagnosis or treatment of diseases, but also after the discovery of promising target molecules, for control of their function or for the purpose of diagnosis. For example, variation in the level of acetylation due to the antibody may be monitored by Western blotting or ELISA, for experimentation comparing that variation with variation in the function of the target molecule. Also, an antibody specific for a desired target molecule may be prepared and an immunological assay system constructed such as sandwich ELISA or RIA in combination with the antibody, to quantify the amount of target molecule in its modified state, as an effective means for disease diagnosis, for example.

### Examples

The present invention will now be explained in greater detail by examples.

### Example 1. Preparation of anti-acetyllysine mouse monoclonal antibody-producing hybridoma ACK2F12

The hybridoma ACK2F12 producing an anti-acetyllysine monoclonal antibody having the light chain variable region amino acid sequence listed in SEQ ID NO: 2 and the heavy chain variable region amino acid sequence listed in SEQ ID NO: 4, was prepared by the following method.

The antigen used for immunization was chemically acetylated Keyhole Limpet Hemocyanin (hereinafter, "KLH"). Acetylated KLH was prepared by the following method. After dissolving 10 mg of KLH in 1 mL of boric acid buffer (20 mM Na₂B₄O₇, pH 9.3), 50 µl of 10 N NaOH and 22.6 µl of acetic anhydride were added while cooling on ice, and incubation was performed for 30 minutes. The solvent was exchanged with PBS using a gel filtration column (PD-10, Amersham) and used for immunization.

Mouse immunization was accomplished in the following manner. The acetylated KLH solution was mixed with TiterMax Gold (CytRx) at 1:1, and repeatedly passed through a joint comprising two syringes joined at a cross-flow joint to form an emulsion. The prepared antigen-containing emulsion was then injected subcutaneously or intraperitoneally at intervals of 1-4 weeks for three immunizations. The antigen amount for each immunization was 50-100 µg. Bovine serum albumin (hereinafter, "BSA") acetylated in the same manner as KLH was solid-phased on an ELISA plate, and after confirming increase in the blood antibody titer by the method described hereunder, acetylated KLH dissolved in PBS was injected intraperitoneally 4 days before myeloma fusion and in the caudal vein 3 days before (dosage: 100 µg/mouse).

Measurement of the antibody titer was accomplished in the following manner. Bovine serum albumin acetylated by the same method as KLH was dissolved in PBS to a concentration of 10 µg/ml, and added to each well of a 96-well ELISA plate at a volume of 50 µl per well, for adsorption overnight at 4°C. After rinsing each well with 0.05% Tween 20-containing PBS (PBST), it was used for the assay. Blood was taken from the caudal vein, and after 30-fold dilution with PBST, it was centrifuged. The obtained supernatant was prepared into a dilution series with PBST, and added at 50 µl to each well of an ELISA plate coated with the acetylated BSA. After 30 minutes of reaction at room temperature, rinsing was performed with PBST, and horseradish peroxidase (HRP)-labeled anti-mouse IgG solution appropriately diluted with PBST was added at 50 µl to each well. After further reaction for 30 minutes at room temperature, ortho-phenylenediamine was used as the substrate for measurement of absorbance at 492 nm to evaluate the activity.

Fusion of the antibody-producing cells and myeloma cells was carried out in the following manner. The spleens were removed three days after intravenous administration of antigen, and the spleen cells were isolated. Polyethylene glycol was used for fusion of 5 x 10⁷ of these cells with separately cultured 1 x 10⁷ mouse myeloma SP2/0-Ag14 cells. The successfully fused cells were selectively cultured in HAT (hypoxanthine/aminopterin/thymidine) medium. Half of the medium was exchanged every few days for about 11 days while culturing was continued, and then the antibody titer of the culture supernatant was measured. The positive well cells were cloned by the limiting dilution method to obtain hybridoma ACK2F12 (deposit number: FERM BP-10351) producing the target antibody. The isotype of the produced monoclonal antibody was IgG1κ.

### Example 2. Measurement of reactivity of antibody for acetylated protein

In order to confirm that the established monoclonal antibody reacts with acetylated protein, the ELISA method was used to examine the reactivity for solid phased acetylated BSA and non-acetylated BSA. PBS-diluted acetylated BSA or non-acetylated BSA at 10 µg/ml concentration was added to each well of an ELISA plate at a volume of 50 µl/well, and after adsorption overnight at 4°C, it was rinsed with PBST. Each purified antibody was subjected to serial dilution with PBST, 50 µl thereof was added to each well, and reaction was performed for 30 minutes at room temperature.

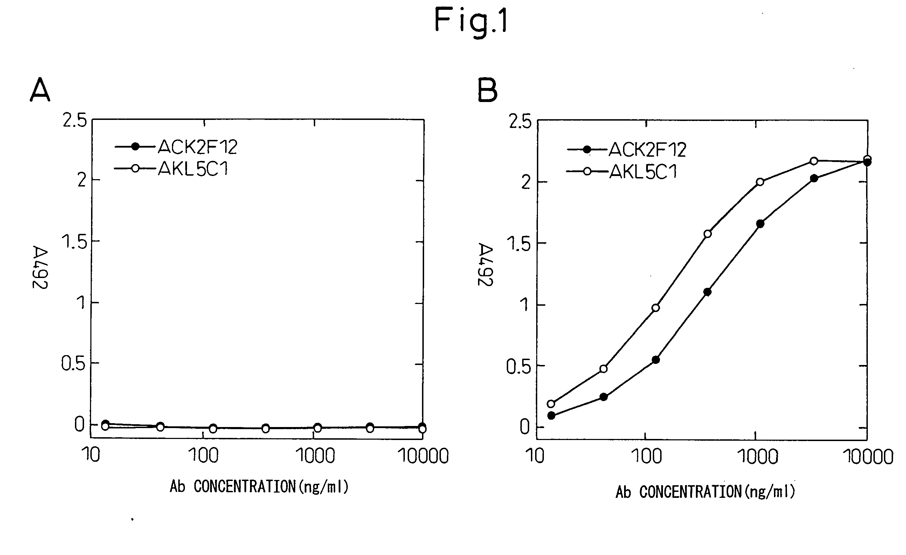

After rinsing with PBST, reaction was performed with HRP-labeled anti-mouse IgG antibody as the secondary antibody, and o-phenylenediamine (OPD) was used as substrate to monitor the amount of bound antibody. The results are shown in Fig. 1. For comparison, results are also shown for the previously reported pan-reactive anti-acetyllysine mouse monoclonal antibody AKL5C1. As shown in Fig. 1, these two antibodies exhibited absolutely no reaction for non-acetylated BSA, while exhibiting strong reactivity for acetylated BSA. This suggests that both are antibodies that specifically react with acetylated proteins.

### Example 3. Competition for ELISA reaction by acetyllysine

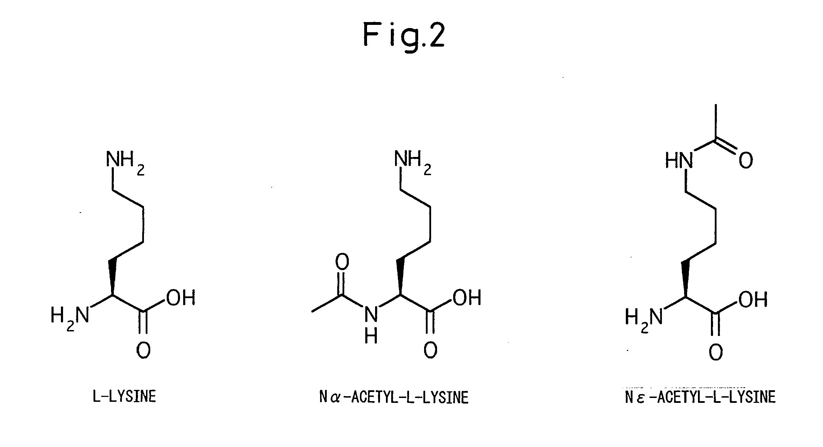

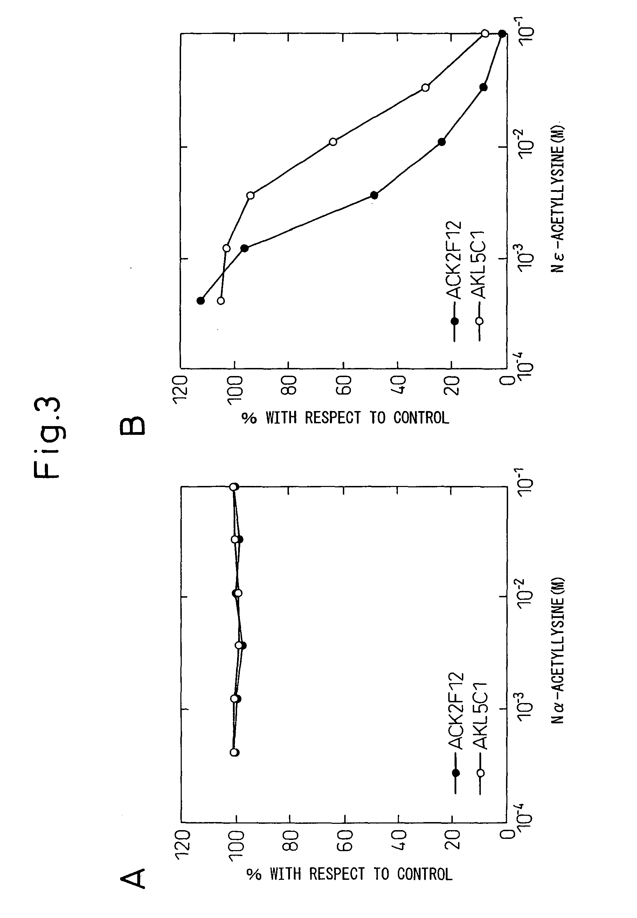

As shown in Fig. 2, two different acetyllysine molecules exist. These two types are Nα-acetyllysine and Nε-acetyllysine. Acetylated BSA was solid-phased by the same method as in Example 2 and reacted with AKL5C1 antibody or ACK2F12 antibody at 1 µg/ml concentration in combination with Nα-acetyllysine or Nε-acetyllysine at different concentrations, yielding the results shown in Fig. 3. As seen in these graphs, both antibodies experienced competition by Nε-acetyllysine, but absolutely no competition by Nα-acetyllysine. These results indicate that the antibodies have a high ability to bind specifically with proteins containing ε-amino group-acetylated lysine.

### Example 4. Reaction of anti-acetyllysine antibody with different acetyllysine-containing peptides

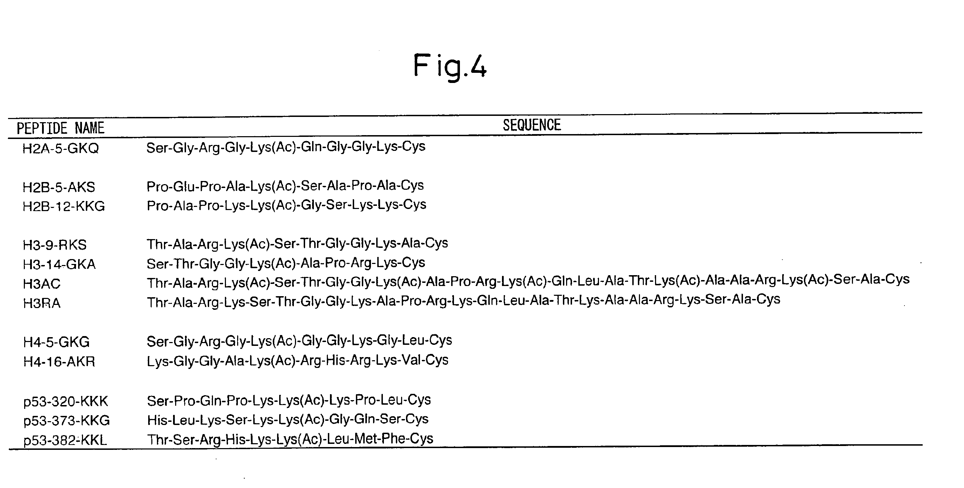

In order to confirm whether or not the antibodies can recognize acetyllysine with minimal dependency on the amino acid sequence surrounding the acetyllysine, the different acetyllysine-containing peptides listed in Fig. 4 were bonded by covalent bonding to an ELISA plate (AquaBind, M & E, Denmark) and the reactivity of each antibody was examined by the same method as Example 2. The peptide bonding was carried out according to the manufacturer's instructions, but the peptide concentration used for bonding was 10 µg/ml.

The antibodies used were, in addition to the anti-acetyllysine mouse monoclonal antibodies AKL5C1 and ACK2F12, also the purportedly pan-reactive commercially available anti-acetyllysine mouse monoclonal antibody AcK-103, and as examples of site-specific antibodies, the two rabbit polyclonal antibodies anti-acetylated histone H3 (Lys-14)(abbreviated as "anti-Ac-H3-K14") and anti-acetylated histone H4 (Lys-16)(abbreviated as "anti-Ac-H4-K16").

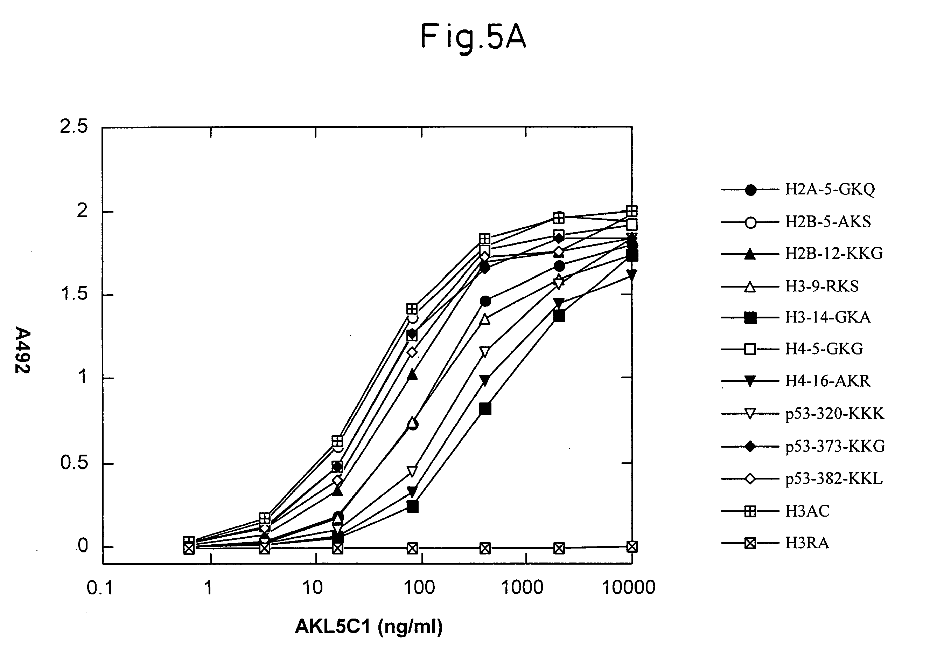

The results are shown in Figs. 5A to 5E. In regard to the three anti-acetyllysine mouse monoclonal antibodies including the commercially available Ac-K-103, ACK2F12 tended to react in a slightly more non-specific manner than the other two antibodies, but exhibited reactivity for all of the acetyllysine-containing peptides studied here. However, none of the antibodies reacted at all with the non-acetylated peptide H3RA.

On the other hand, the two site-specific anti-acetyllysine antibodies reacted most strongly with peptides containing the respective purported acetylation sites.

### Example 5. Comparison of antibody reactivities using MOLT-4F cell lysate

Experimentation by Western blotting was conducted to compare the antibody reactivities. Cells of the human T cell leukemia cell line MOLT-4F were treated for 6 hours with the histone deacetylase inhibitor trichostatin A, at a concentration of 1 µM, and then a cell lysate was prepared. As a control, a trichostatin A-untreated cell lysate was also prepared. Five anti-acetyllysine antibodies were compared as in Example 4: AKL5C1, ACK2F12, Ac-K-103, anti-Ac-H3-K14 and anti-Ac-H4-K-16. The results are shown in Fig. 6. A 6 µg protein equivalent was electrophoresed with 15% SDS-PAGE in each lane, and transferred to a PVDF membrane. HRP-labeled anti-mouse IgG antibody was used as the secondary antibody, and bands were detected with ECL Plus of Amersham.

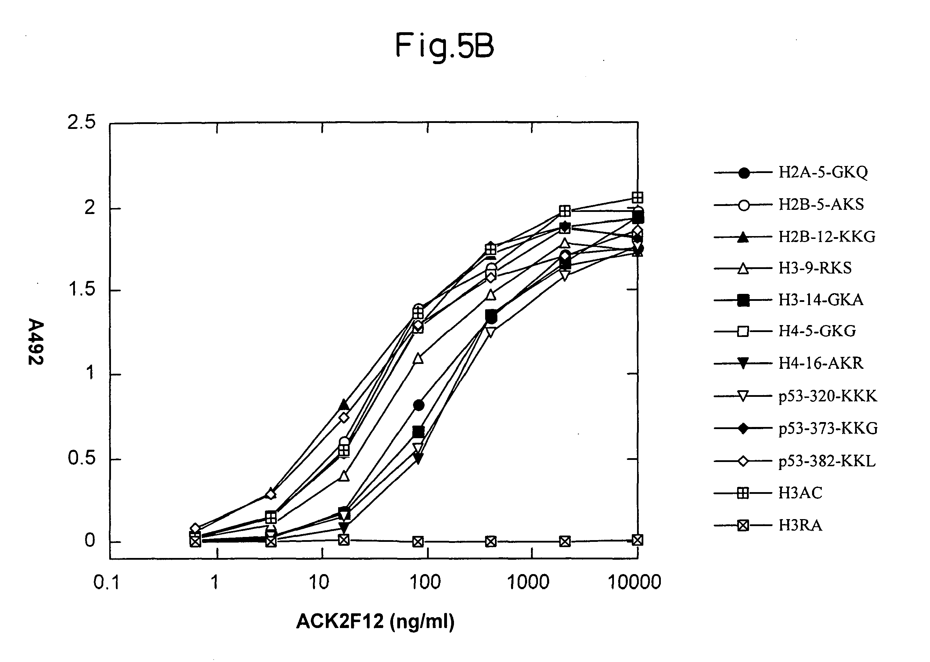

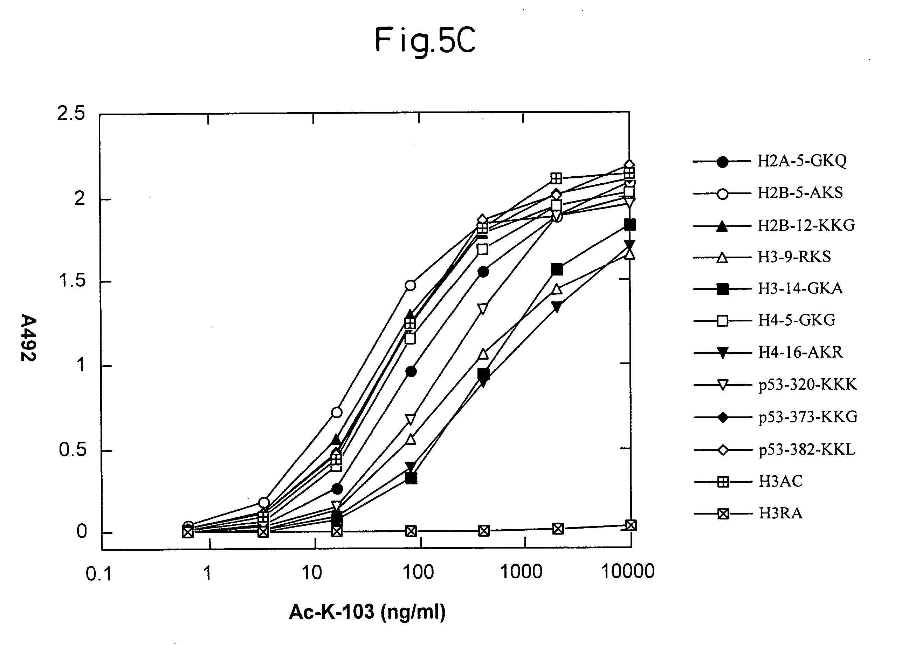

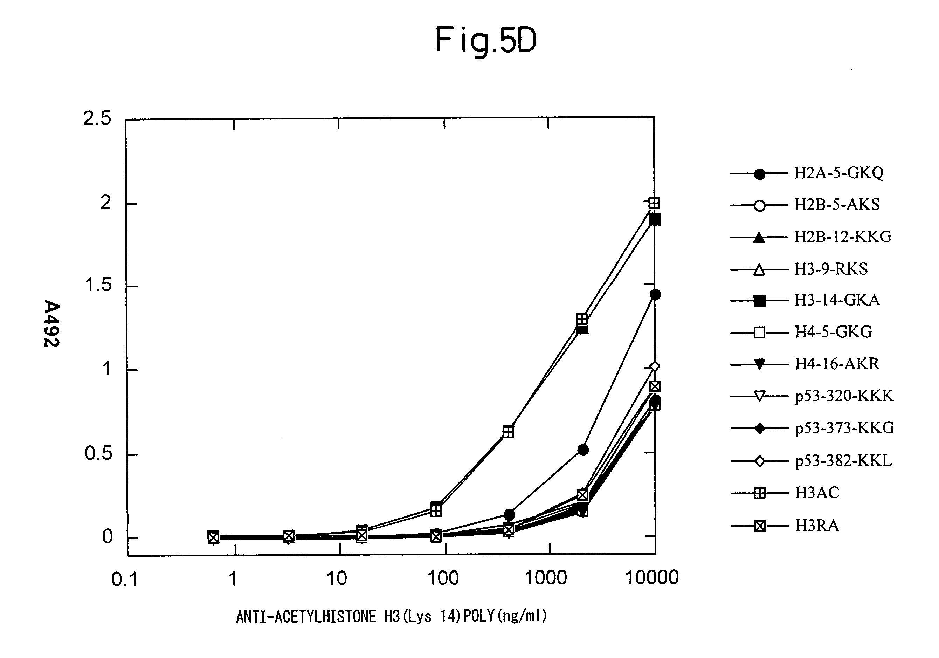

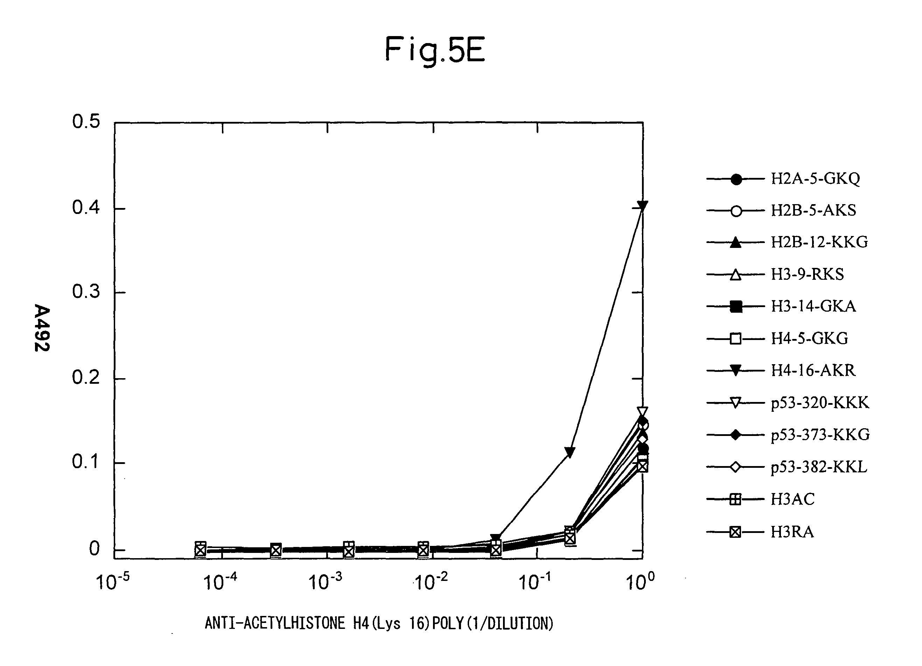

First, when the three mouse monoclonal antibodies are compared, the established ACK2F12 antibody was able to detect the greatest amount of acetyllysine-containing protein. This was followed by AKL5C1, with the poorest detection exhibited by the commercially available Ac-K-103. The reaction results were all obtained with a concentration of 1 µg/ml. While no significant difference was found in the reactivities of the antibodies with respect to the different acetyllysine-containing peptides when examination was by ELISA (Fig. 5), using Western blotting showed a significant difference in detection power. All of the antibodies were able to detect accumulated acetylation of proteins by trichostatin A treatment. Of the two acetylation site-specific antibodies, however, anti-Ac-H3-K14 selectively recognized histone H3 alone, and detected accumulated acetylation of this molecule by trichostatin A treatment. On the other hand, anti-Ac-H4-K16 weakly reacted only with trichostatin-treated histone H4.

### Example 6. Detection of differences in acetylation levels with different cells types, and comparison of antibodies

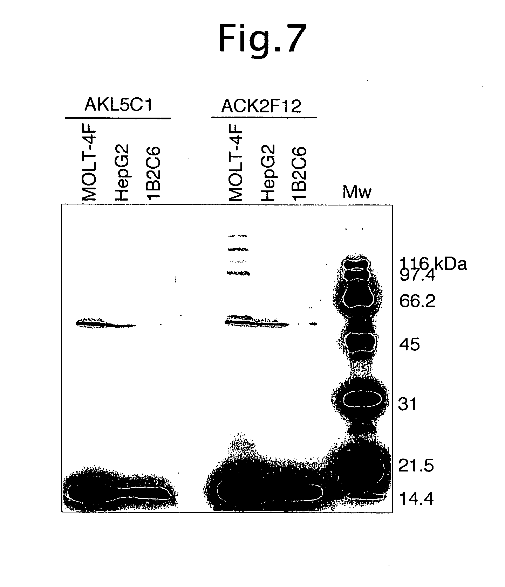

Cell lysates were prepared from the three different cell lines MOLT-4F, HepG2 and 1B2C6, and after electrophoresis at 30 µg per lane, they were transferred to a PVDF membrane. Western blotting was conducted under the same conditions using AKL5C1 and ACK2F12 antibody at a concentration of 1 µg/ml each, in an attempt to detect the basal level of acetylated protein for each cell type. The results are shown in Fig. 7. Similar to the results in Fig. 6, more bands could be detected with ACK2F12 antibody than with AKL5C1 antibody, and therefore the detection power of ACK2F12 in Western blotting was confirmed to be superior.

### Example 7. Enhancement of detection power by direct labeling of anti-acetyllysine antibodies

Since detection of different acetyllysine-containing proteins was found to be possible using the established anti-acetyllysine antibody, it was then attempted to directly label the antibody with a reporter enzyme, with the intention of further enhancing the detection power. Two methods were attempted. The first was a method using EZ-link Plus Activated Peroxidase by Pierce Co. This method employs HRP having aldehyde groups introduced to react with amino groups in antibody molecule. The labeling was carried out according to the manufacturer's protocol. The labeled antibody produced by this method was designated as Ab-HRP-NH₂.

The other method involved weak reduction with cysteamine to cleave the disulfide bonds between the heavy chains, and bonding of maleimide-activated HRP to the exposed free sulfhydryl groups. Specifically, the labeling was accomplished by the following procedure. After dissolving 0.5 mg of antibody in 0.5 ml of 0.1 M sodium phosphate/5 mM EDTA at pH 6, 3 mg of cysteamine was added. Reaction was conducted at 37°C for 1.5 hours, and then the solvent was exchanged with 5 mM EDTA-containing PBS (PBS-EDTA) by gel filtration to obtain reduced antibody. Separately, 3 mg of HRP was dissolved in 1.5 ml of PBS-EDTA, and then 3 mg of sulfo-SMCC (Pierce) was added and reaction was conducted at 37°C for 30 minutes.

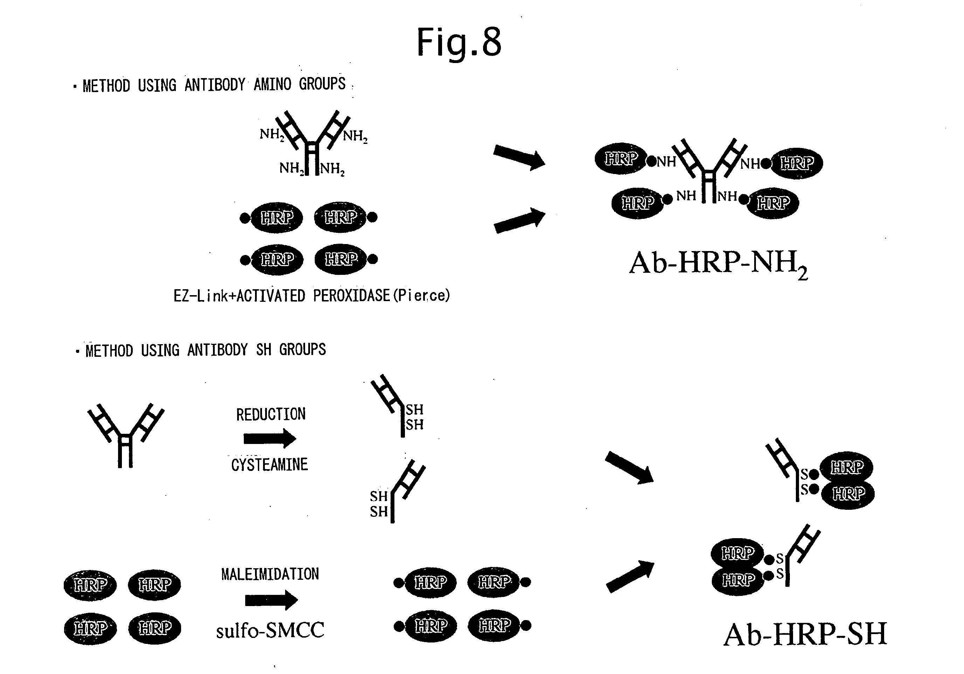

The solvent was exchanged with PBS-EDTA by gel filtration to obtain maleimide-activated HRP. The reduced antibody solution was mixed with the maleimide-activated HRP solution, and the mixture was concentrated to 500 µl with an ultrafiltration membrane with a molecular weight cutoff of 50,000, after which reaction was conducted overnight at 4°C. Upon completion of the reaction, 1 mg of cysteamine was added, reaction was conducted at room temperature for 30 minutes to eliminate the unreacted maleimide, and then the solvent was exchanged with PBS by gel filtration to obtain the labeled antibody. The labeled antibody prepared by this method was designated as Ab-HRP-SH. Fig. 8 is a schematic diagram showing a comparison between these two labeling methods.

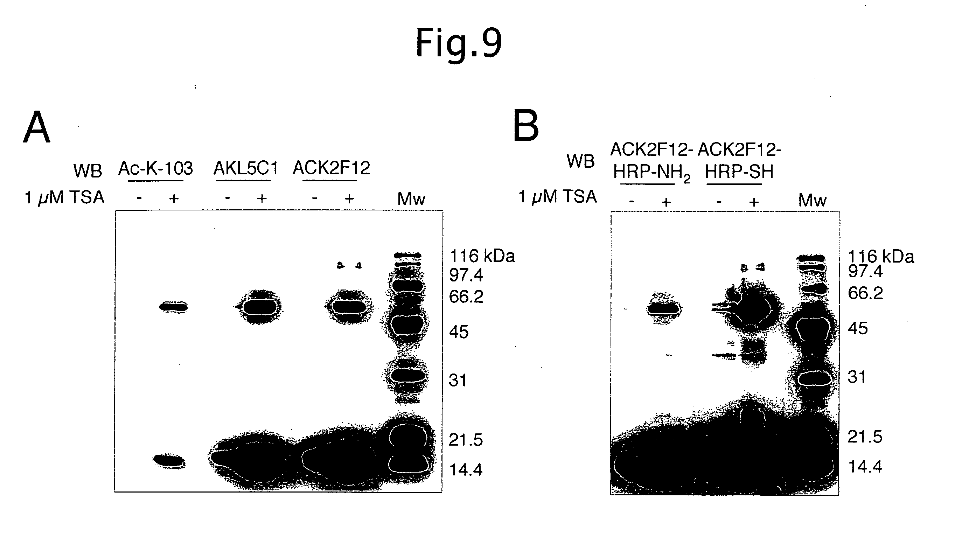

MOLT-4F cell lysate prepared by the same method described in Example 5 was used to compare the detection powers of these labeled antibodies. The results are shown in Fig. 9. Trichostatin A-treated or -untreated MOLT-4F cell lysate was subjected to electrophoresis on 12% SDS-PAGE gel at 2 µg/lane, and was then transferred to PVDF. Each detection was carried out using antibody at a concentration corresponding to 1 µg/ml. Image A in Fig. 9 shows the results for the method using the unlabeled antibody, and similar to the results of Fig. 6, it indicates that ACK2F12 had the higher detection power.

Image B in Fig. 9 shows the results for detection of the same sample with HRP-labeled ACK2F12 antibody, and clearly more bands were detected than with the unlabeled antibody, indicating that the directly labeled anti-acetyllysine antibody is able to detect more acetyllysine-containing protein. This effect was particularly notable when HRP was bound via sulfhydryl groups. It therefore suggests that the amino groups of the antibody molecules may play an important role in acetyllysine recognition by the antibodies.

### Example 8. Detection of chemically acetylated BSA by ACK2F12-HRP-SH

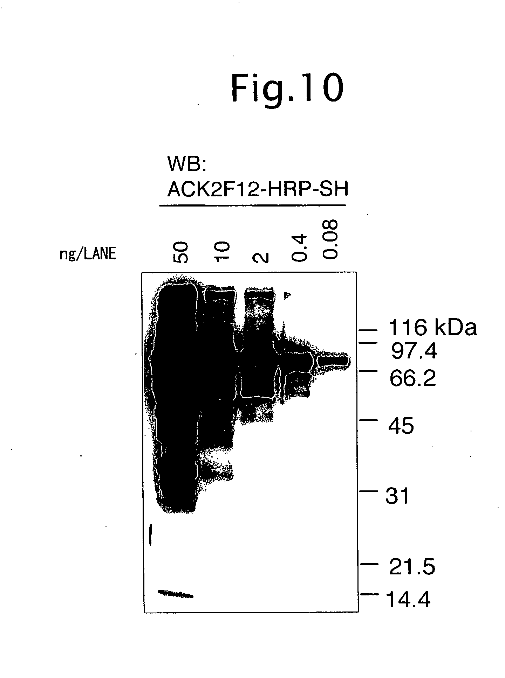

Detection of chemically acetylated BSA with ACK2F12-HRP-SH was carried out by Western blotting to examine how small an amount of modified protein can be detected. The results are shown in Fig. 10. Acetylated BSA prepared by the method described in Example 1 was subjected to electrophoresis in amounts per lane from 50 ng/lane maximum to 80 pg/lane minimum, and after transfer to a PVDF membrane, it was detected with ACK2F12-HRP-SH labeled antibody. As shown in the photograph, the chemically modified BSA could be sufficiently detected even in a trace amount of 80 pg. As the chemically modified BSA is thought to be acetylated at multiple sites, direct extrapolation for detection of intact protein thought to be acetylated at only one to a few sites per molecule is not possible, but these results do indicate high detection power for the modified antibody.

### Example 9. Detection of acetyllysine-containing protein in mouse organ lysates

Since a system was established allowing high-sensitivity detection of acetyllysine-containing protein, it was used in an attempt to detect acetyllysine-containing proteins in mouse organ lysates. Organs from Balb/c mice (female) were ultrasonically disrupted in 9.8 M urea, 4% CHAPS, 100 mM dithiothreitol (DTT) and centrifuged, and the supernatants were used for analysis. Each organ lysate was electrophoresed on 12% gel with 30 µg of protein per lane and then transferred to a PVDF membrane and subjected to Western blotting with ACK2F12-HRP-SH.

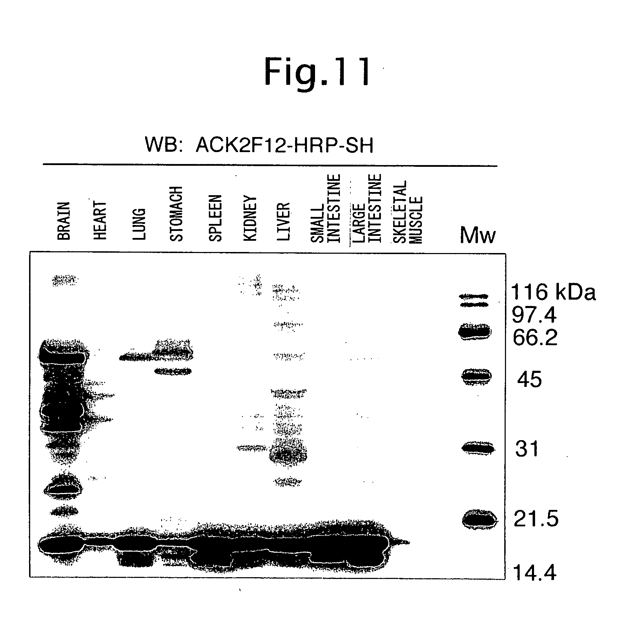

The sample prior to electrophoresis was diluted with 130 mM Tris-HCl, pH 6.8, 4% SDS, 15% glycerol, 80 mM DTT, 0.01% bromophenol blue, and treated at 37°C for 30 minutes. The results are shown in Fig. 11. A highly characteristic staining pattern was exhibited by each organ, and very deeply stained bands appeared for the brain. Characteristic bands were also seen for the lung, stomach, liver, etc. Distinct bands were found at the histone-attributed low molecular weight region (14.4-21.5 K) for almost all of the organs, but none was seen for the skeletal muscle.

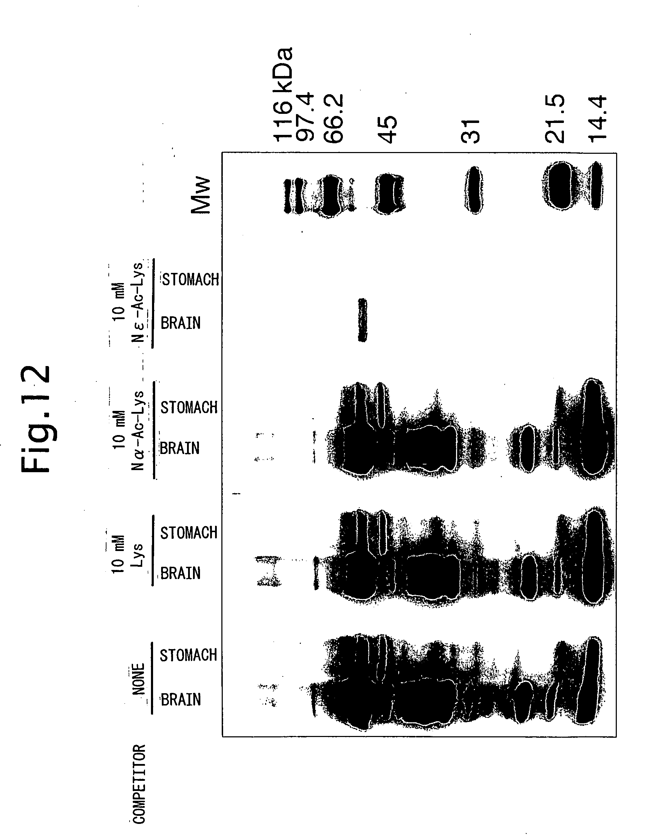

Next, in order to confirm that the detected bands were acetyllysine-containing protein and that they could be specifically recognized by anti-acetyllysine antibody, the brain and stomach lysates of the mouse organ lysates were subjected to electrophoresis at 15 µg per lane and transferred to a PVDF membrane by the same method, after which the labeled antibody combined with 10 mM lysine, Nα-acetyllysine and Nε-acetyllysine was used for a competitive binding experiment. The results are shown in Fig. 12. Since most of the bands detected by Western blotting only disappeared in the presence of Nε-acetyllysine, it was strongly suggested that these bands appeared not as a result of non-specific binding of the antibody but rather were specifically detected, or in other words, that these represented acetyllysine-containing proteins. It is believed, therefore, that using the labeled antibody of the invention allows efficient detection of new acetyllysine-containing proteins.

### Example 10. Sequence determination of ACK2F12 antibody variable region

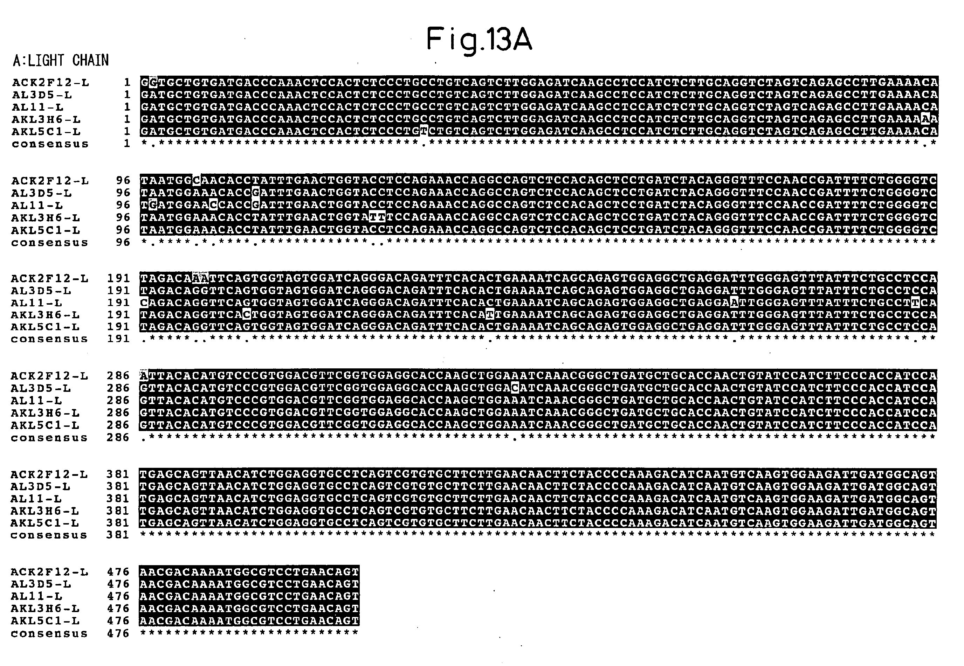

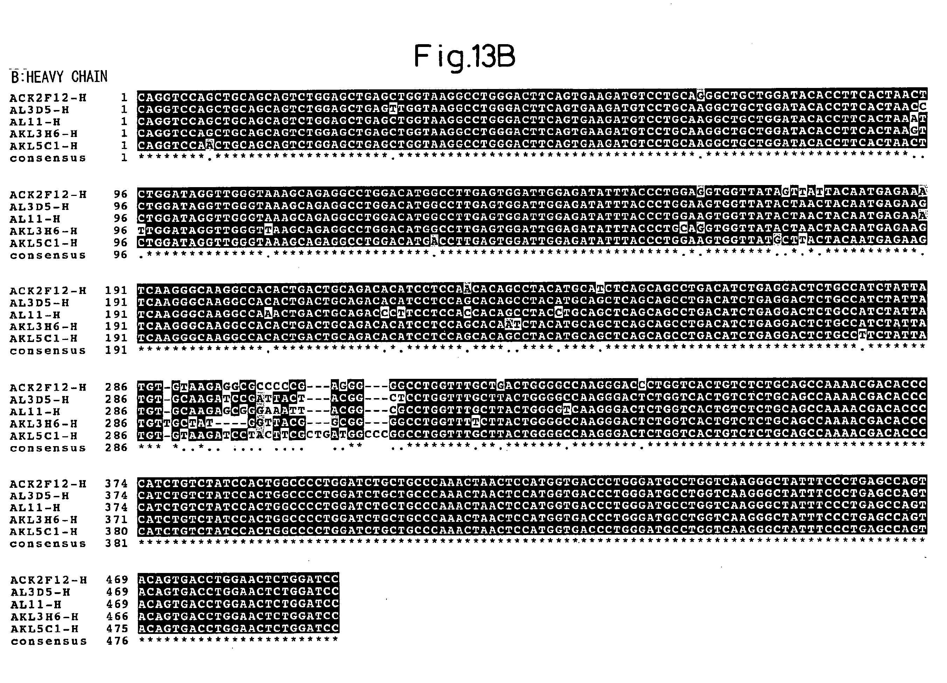

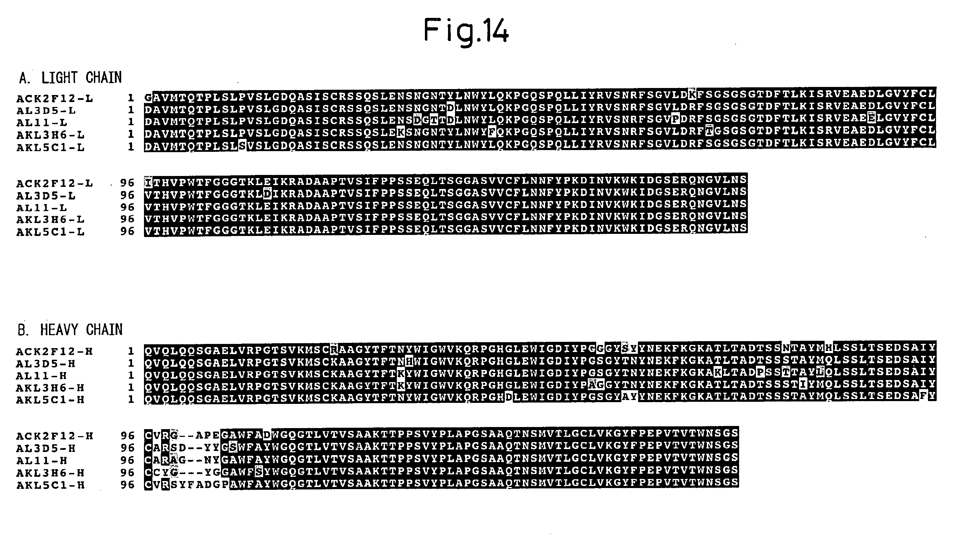

We have indicated that ACK2F12 antibody has superior properties to previously reported pan-reactive anti-acetyllysine antibodies, but in order to examine what differences in molecular structure of the antibody are responsible for the difference in properties, we cloned the variable regions of the antibody according to a known method and determined the nucleotide sequences of the cDNA coding for the variable regions of the light and heavy chains. The results are shown in Fig. 13A and Fig. 13B, along with the previously reported anti-acetyllysine antibody variable region cDNA sequences. The amino acid sequences predicted from the cDNA sequences are shown in Fig. 14 together with the reported antibody sequences. The amino acid sequences corresponding to the cDNA sequences of the light chain and heavy chain variable regions of ACK2F12 antibody are listed as SEQ ID NOS: 1 and 3, respectively.

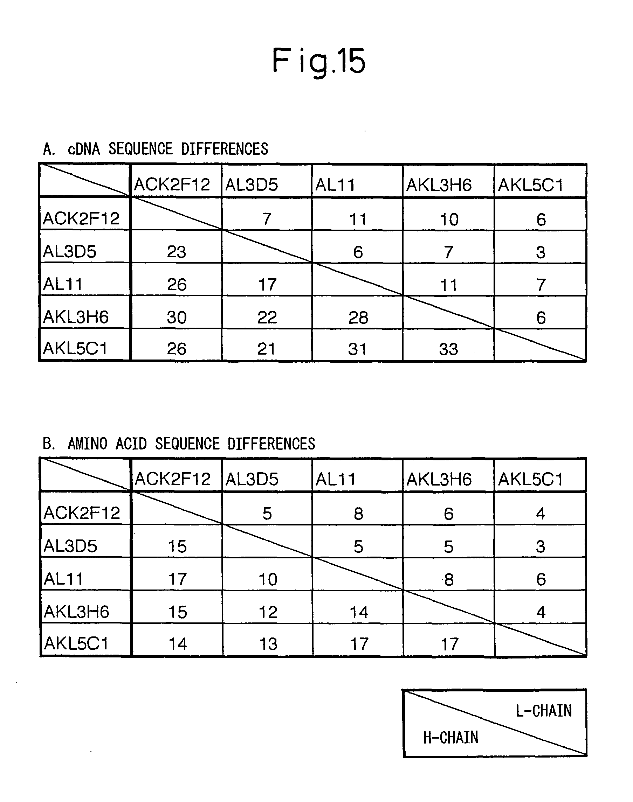

As shown in these alignments, ACK2F12 antibody has basically the same framework sequence as the four different antibodies whose sequences have been reported to date. However, the sequences are not identical, with differences in at least 6 locations of the light chain and at least 24 locations of the heavy chain for the nucleotide sequence, and in at least 4 locations of the light chain and at least 14 locations of the heavy chain for the amino acid sequence, compared to the reported antibodies. The results for mismatches between the sequences are summarized in Fig. 15. These results indicate that the ACK2F12 antibody established in our research has a basic framework sequence similar to the reported sequences, but that the sequences have several differences which may be responsible for the high detection power of the antibody.

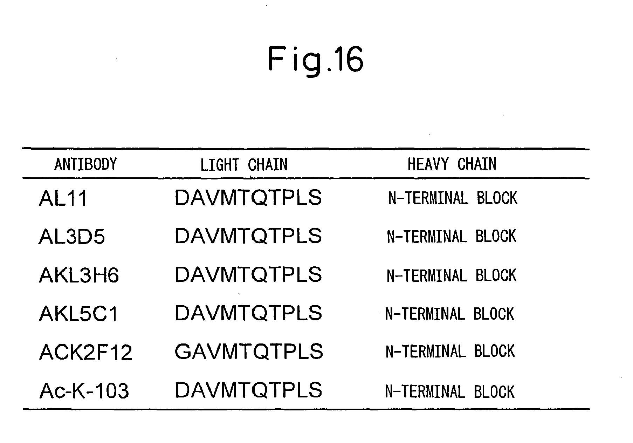

Also, while no hybridoma was available for the marketed Ac-K-103 antibody, it was developed by electrophoresis and transferred to a PVDF membrane, the heavy and light chains were cleaved and the N-terminal sequences were determined with a protein sequencer (Beckman, LF-3000). AL11, AL3D5, AKL3H6 and AKL5C1 antibodies were also examined by the same method, yielding the results shown in Fig. 16. As seen in this table, the N-terminal sequence of the L chain of Ac-K-103 antibody was DAVMTQTPLS, which completely matched the N-terminal sequence of the other antibodies except for ACK2F12. None of the heavy chain N-terminal sequences could be determined for any of the antibodies. This suggests that all of the N-termini of the antibodies were blocked.

These results therefore indicated that mouse monoclonal antibodies that recognize acetyllysine residues with low surrounding sequence dependency have essentially the same the framework sequence. However, it was also found that differences in the variable region sequences have major effects on the properties of the antibodies including detection power.

### Industrial Applicability

The anti-acetyllysine monoclonal antibody and enzyme-labeled antibody provided by the invention can be used to search for new acetyllysine-containing proteins as possible disease-associated factors or diagnostic markers. The anti-acetyllysine monoclonal antibody and enzyme-labeled antibody provided by the invention are also useful for control of biomolecular functions in which lysine residue acetylation is strongly implicated, or for disease diagnosis based on detection of acetyllysine-containing proteins.

## Claims

1. A mouse monoclonal antibody whose light chain variable region amino acid sequence is specified as the sequence listed as SEQ ID NO: 2 or as that sequence with 2 or 3 different amino acids, and the heavy chain variable region is specified by the sequence listed as SEQ ID NO: 4 or as that sequence with 2 or 3 different amino acids.

2. A mouse monoclonal antibody according to claim 1, which recognizes Nε-acetyllysine.

3. A mouse monoclonal antibody according to claim 1, wherein the light chain variable region amino acid sequence is specified as SEQ ID NO: 2, and the heavy chain variable region amino acid sequence is specified as SEQ ID NO: 4.

4. A mouse monoclonal antibody according to claim 3, which recognizes Nε-acetyllysine.

5. A mouse monoclonal antibody according to claim 4, which is produced by the mouse hybridoma designated as deposit number FERM BP-10351.

6. An anti-acetyllysine labeled antibody obtained by reducing an anti-acetyllysine antibody and binding a labeling molecule to the exposed sulfhydryl groups.

7. An anti-acetyllysine labeled antibody according to claim 6, wherein the labeling molecule is horseradish peroxidase.

8. An anti-acetyllysine labeled antibody according to claim 6 or 7, wherein the anti-acetyllysine antibody prior to labeling is an anti-acetyllysine antibody according to claim 2.

9. An anti-acetyllysine labeled antibody according to claim 6 or 7, wherein the anti-acetyllysine antibody prior to labeling is an anti-acetyllysine antibody according to claim 4.

10. An anti-acetyllysine labeled antibody according to claim 6 or 7, wherein the anti-acetyllysine antibody prior to labeling is an anti-acetyllysine antibody according to claim 5.
